# EUROPÄISCHE PATENTSCHRIFT

(11) **EP 0 713 094 B1**
(45) Veröffentlichungstag und Bekanntmachung des Hinweises auf die Patenterteilung: **13.02.2002**
(21) Anmeldenummer: 95116177.7
(22) Anmeldetag: 13.10.1995
(51) Int. Cl.: G01N 33/539, G01N 33/541, G01N 33/537

(54) **Heterogener Immunoassay mittels präzipitierbarer Festphase**
Heterogeneous immunoassay with precipitable solid phase
Immunoessai hétérogène avec une phase solide précipitable

(30) Priorität: 12.11.1994 DE 4440487
(43) Veröffentlichungstag der Anmeldung: 22.05.1996
(73) Patentinhaber: Dade Behring Marburg GmbH, 35001 Marburg (DE)
(72) Erfinder: Kraus, Michael, Dr., D-35041 Marburg (DE)

(56) Entgegenhaltungen:
- EP-A- 0 044 988
- EP-A- 0 568 797
- US-A- 4 720 465
- CLINICAL CHEMISTRY, Bd. 30, Nr. 9, September 1984 WINSTON US, Seiten 1457-1461, RATTLE ET AL. 'New separation method for ....'

## Beschreibung

Die Erfindung bezieht sich auf ein Verfahren zur Durchführung heterogener Immunoassays, insbesondere auf eine Methode zur Trennung einer beschichteten Festphase von einer flüssigen Phase durch Fällung und anschließender Zentrifugation, wobei eine nachweisbare Aktivität in der flüssigen Phase verbleibt.

Immunchemische Verfahren werden häufig zum Nachweis von Analyten z. B. Antikörpern angewendet, die in biologischen Flüssigkeiten nur in geringen Konzentrationen vorhanden sind, deren Nachweis aber wichtig für die Diagnose und Therapie von Erkrankungen ist.

Bei immunologischen Bestimmungsverfahren unterscheidet man zwischen homogenen und heterogenen Verfahren. Bei homogenen Verfahren erfolgt vor der Nachweisreaktion keine Trennung der Bindungspartner, während bei heterogenen Verfahren Bindungs- und Nachweisreaktion nacheinander, nach physikalischer Trennung der Bindungs- und Nachweispartner, erfolgen. Homogene Nachweisverfahren, wie etwa die Messung von Trübungen bei Partikel-verstärkten Agglutinationsverfahren, sind oft schneller in der Durchführung, da aber der Trennungsschritt fehlt, sind sie oft störanfälliger und weniger sensitiv als heterogene Verfahren.

Weiterhin unterscheidet man zwischen direkten und kompetitiven Nachweisverfahren. Bei direkten Nachweisverfahren erfolgt die Bindungs- und Nachweisreaktion am Analyten, etwa durch Bindung an einen ersten Antikörper an der Festphase und durch Bindung eines , ein nachweisbares Prinzip tragendes Konjugat eines zweiten Antikörpers an diesen Komplex, z. B. Sandwichassays. Bei kompetitiven Nachweisverfahren hingegen tritt der Analyt der Probe mit einem markierten Analyten oder einer Analyt-ähnlichen Verbindung in Konkurrenz um die Bindung an eine Festphase, die z. B. mit einem analytspezifischen Antikörper beschichtet sein kann. Der Nachweis der Konkurrenz kann dabei in einem homogenen System, z.B. durch Inhibierung der Agglutinationsreaktion bzw. in einem heterogenen System, z.B. durch Nachweis der gebundenen Menge des markierten Analyten an der Festphase, erfolgen. Solche kompetitiven Verfahren werden vorteilhafterweise zum Nachweis von Analyten mit nur einer spezifischen Bindungsstelle oder bei sehr kleinen Molekülen (Haptenen) verwendet, die nur eine Bindungsreaktion und nicht wie bei direkten Nachweisverfahren notwendig zwei Bindungsreaktionen (Festphase und Konjugat) erlauben.

Der Nachweis von Proteinen mit nur einem charakteristischen Epitop stellt, ebenso wie der Nachweis von Haptenen, besondere Anforderungen an den Testaufbau. So sind homogene Immunoassays etwa nach dem Agglutina-tionsprinzip allenfalls in einem kompetitiven Aufbau möglich. Die, wie eingangs diskutiert, meist geringe Sensitivität dieser Assays erfordert heterogene Verfahren. Herkömmliche immunologische, heterogene Verfahren benutzen zur Trennung von gebundenem und noch in Lösung befindlichem Analyten bzw. Analyt-Konjugat zum Analyten komplementäre Bindungspartner, die auf einem Träger, wie z. B. Röhrchen oder Mikrotitrationsplatten immobilisiert wurden und daher gewaschen werden können. Im Zuge der Weiterentwicklung von heterogenen Verfahren ist die Verwendung pipettierbarer Festphasen vorteilhaft, da diese die Bestimmung verschiedener Analyten in verschiedenem Umfang erlauben ('random access'). Als pipettierbare Festphasen werden z. B. beschichtete Partikel verwendet.

So ist die Verwendung von an Agarosepartikel gekoppelten Antikörpern in der Immunchromatographie zur immunchemischen Isolierung von Analyten z. B. in der DE 41 26 436 beschrieben worden . Daneben sind weitere Verfahren beschrieben, bei dem beschichtete Mikropartikel zur Trennung von Analyten verwendet werden. Diese Verfahren unterscheiden sich in der Art und Weise wie die Trennung der beschichteten Partikel erfolgt.

Beschrieben worden ist auch schon, z. B. in der DE 41 24 778 die Trennung durch Filtration der Mikropartikel-Suspension in der zu untersuchenden Lösung. In ähnlicher Weise kann auch bei einem kompetitiven Immunoassay verfahren werden. Bestimmt wird entweder die an der Festphase verbliebene Menge des Analyten oder in kompetitiven Verfahren die im Filtrat verbleiben-den freien Analyt/Enzym-Konjugate. In einer weiteren Variante werden die in den Immunkomplexen verbliebenen Analyt/ Enzym-Konjugate wieder freigesetzt und die Aktivität des Enzyms bestimmt.

Beschrieben wurde ferner auch, daß beschichtete Partikel, nachdem sie mit der Analyt-haltigen Lösung in Kontakt gebracht wurden, manuell abgetrennt und vor der weiteren Analyse des gebundenen Analyten gewaschen werden.

Z. B. in US (91/716,144) wird beschrieben, daß beschichtete Magnetpartikel durch die Annäherung von Magneten immobilisiert und so vor der weiteren Analyse des gebundenen Analyten gewaschen werden können.

Alle diese beschriebenen Verfahren erfordern spezifisch auf die Trennsysteme abgestellte Geräte. Es bestand daher ein Bedarf für ein Verfahren zur Trennung der Festphase, das auch auf gängigen klinisch-chemischen Analyzern ohne spezielle Wasch- und/oder Trennvorrichtungen applizierbar ist.

In JP 86-230925 wird der Analyt in Gegenwart von Analyt/Enzym-Konjugat an Antikörper beschichtete Polystyrolpartikel gebunden, die mittels Zentrifugation aus der Reaktionslösung abgetrennt und gewaschen werden. Die Enzymaktivität des Präzipitats wird bestimmt. Diese Methode erfordert jedoch sehr hohe Zentrifugalkräfte und ist daher nicht an gängigen Zentrifugalanalysatoren anwendbar. Zudem erfordert das beschriebene Verfahren einen separaten Waschschritt. Gängige Zentrifugalanalysatoren arbeiten mit maximalen Beschleunigungen von bis zu etwa 1500 x g.

In JP 88-143257 wird in einem direkten Verfahren ein Gemisch aus gegen den Analyten gerichteten, beschichteten Partikeln und Antikörper-Konjugat gebildet und dies mit der Analyt-haltigen Lösung über mehrere Stunden bei Raumtemperatur stehen gelassen. Anschließend wird aus dem Überstand verbliebene, lösliche Aktivität des Antikörper-Konjugats separat bestimmt. Anscheinend, die Verfahrensbeschreibung geht hierauf nicht ein, sedimentieren während der langen Inkubationszeit die entstehenden Komplexe aus Partikel-Analyt-anti-Analyt/Konjugat, so daß im Überstand das anti-Analyt/ Konjugat entsprechend der Menge an freiem Analyt abgereichert wird. Diese Methode ist äußerst unbefriedigend, da die Sedimentationsdauer äußerst lange Inkubationszeiten erfordert und durch mechanische Erschütterungen bzw. durch Schwankungen in der Eintauchtiefe des Pipettierarms auch diese Konjugat-haltigen Komplexe mit in die Konjugat-Bestimmung gelangen können.

In GB 84-11706 erfolgt die Trennung eines Gemisches aus Analyt (ein Hapten), anti-Analyt-Antikörper/Label 1-Konjugat und Analyt/Label 2-Konjugat derart, daß in einer ersten Inkubation Komplexe aus dem anti-Analyt-Antikörper/Label 1-Konjugat mit dem freien Analyten bzw. kompetitiv mit dem Analyt/Label 2-Konjugat entstehen. Die Trennung des gebundenen Analyt/Label 2-Konjugats geschieht in einem weiteren Schritt durch Immobilisierung an eine Festphase durch Bindung des Label 1. Zur Anwendung kommen hierzu insbesondere Röhrchen beschichtet mit gegen Label 1 gerichteten Antikörpern. In einer anderen Variante wurde zunächst Antikörper gegen Label 1 zugesetzt und dann mit Antikörper gegen diese anti-Label 1 gerichteten Antikörper beschichtetes Kaolin. In dieser zweiten Variante erfolgte die Abtrennung mittels Zentrifugation. Die Aktivität des Labels 2 wird dann im Präzipitat nachgewiesen, das um entsprechende Verschleppung aus der Lösung zu eliminieren, zusätzlich gewaschen werden muß. Dieses Verfahren ist sehr komplex. So werden in der zweiten Variante Antikörper von 3 verschiedenen Spezies eingesetzt. Demgemäß.summieren sich die benötigten Inkubationszeiten auf mehrere Stunden. Dieses Verfahren ist zudem auf den Nachweis von Haptenen beschränkt.

Obwohl die bisher beschriebenen Verfahren z. T. auch schon in der Praxis eingesetzt wurden, hat es sich gezeigt, daß keines der üblichen Verfahren optimal für den Einsatz an gängigen Zentrifugalanalysatoren geeignet ist.

Der vorliegenden Erfindung lag somit das technische Problem zugrunde, ein heterogenes immunchemisches Nachweisverfahren zu finden, das zur Verwendung an einem der gängigen Zentrifugalanalysatoren geeignet ist.

Die Lösung dieses technischen Problems erfolgt durch die Bereitstellung der in den Patentansprüchen beschriebenen Ausführungsformen.

Überraschenderweise wurde festgestellt, daß ein solches Verfahren möglich ist, wenn nach der Inkubation der Probe mit einem immobilisierten spezifischen Bindungspartner und einer markierten spezifischen Nachweissubstanz die Festphase mittels Zugabe einer Substanz aus der Gruppe, die folgende Substanzen umfaßt:
i) ein gegen die Festphase gerichteter spezifischer Bindungspartner,
ii) ein gegen die nachzuweisende Substanz gerichteter Bindungspartner,
iii) ein gegen eine auf der Festphase immobilisierte Ankersubstanz gerichteter Bindungspartner,
   - wobei die Antikörper bevorzugterweise von einer anderen Spezies sind als der unmarkierte spezifische Bindungspartnergefällt wird, die Festphase bei Beschleunigungen von bevorzugterweise 10 bis 1000 x g, besonders bevorzugterweise 200 bis 800 x g in einem gängigen Zentrifugalanalysator abzeritrifugiert wird und zum Nachweis die Konzentration der unverbrauchten markierten spezifischen Nachweissubstanz im Überstand bestimmt wird.

Die erfindungsgemäße Wirkung beruht wahrscheinlich darauf, daß die Ausfällung der Partikel durch Zugabe von für diese Partikel affinen "Bindungspartnern" (siehe Beispiel 4) die Sedimentationsrate so erhöht, daß eine Abtrennung an einem gängigen Zentrifugalanalysator in einer kurzen Zeit etwa 0.1 - 10 min, bevorzugter-. weise 1 - 3 min erfolgen kann. Die geeignete Abstimmung der Konzentrationen der Bindungspartner z. B. in Hinblick auf die Vermeidung von "high-dose-hook"-Effekten kann der Fachmann mittels geeigneter Versuche leicht durchführen.

Eine Verbesserung der Nachweisempfindlichkeit kann durch die Verdünnung der Latexsuspension eingestellt werden (Beispiel 5). Da aber entsprechend die Analyt/Enzym-Konjugat Konzentration reduziert wird, kann es notwendig werden, die im Überstand befindliche Enzymaktivität indirekt, d.h. unter Verwendung weiterer nachgeschalteter Reaktionen zu bestimmen (Beispiel 6).

Das erfindungsgemäße Verfahren zeichnet sich durch die geringere Anzahl Pipettierschritte, weniger notwendige Komponenten und vor allem durch eine deutlich kürzere Abarbeitungszeit aus, die normalerweise im Bereich von 1 bis 60 min, bevorzugterweise im Bereich von 10 bis 30 min liegt. So werden im erfindungsgemäßen Verfahren nur Antikörper von 2 Spezies benötigt . Weiterhin erfolgt im erfindungsgemäßen Verfahren die Bestimmung direkt aus dem Überstand. Zudem erfordert dieses Verfahren keine speziellen Wasch- und Trennvorrichtungen und ist daher auf die gängigen klinisch-chemischen Analysatoren übertragbar. Weiterhin erlaubt dieses Verfahren sowohl die Anwendung in kompetitiven als auch in direkten Nachweisverfahren. Schließlich ist dieses Verfahren sowohl für die Bestimmung von Haptenen, Substanzen mit nur einem spezifischen als auch für den Nachweis von Substanzen mit mehreren spezifischen Epitopen anwendbar und somit wesentlich vielseitiger als die bisher beschriebenen Verfahren.

Markierte spezifische Nachweissubstanzen sind, in Abhängigkeit von dem Bestimmungsverfahren, bei kompetitiven Assays markierte Analyten und bei Sandwichassays markierte spezifische Bindungspartner.

Markierte spezifische Bindungspartner sind spezifische Bindungspartner, die entweder direkt eine nachweisbare Markierung tragen oder eine Gruppe, über die eine Markierung oder eine Nachweisreaktion angekoppelt werden kann. Markierte Analyten im Sinne der Erfindung sind z. B. Analyten, Analytderivate und Analytanaloge, die entweder direkt eine nachweisbare Markierung tragen oder eine Gruppe über die eine Markierung angekoppelt werden kann.

Markierungen im Sinne der Erfindung können z. B. sein, Enzyme, Isotopen, fluorogene oder chemolumineszente Gruppen aber auch gefärbte oder farbige Partikel.

Spezifische Bindungspartner im Sinne der Erfindung sind z. B. anti-Analyt Antikörper, spezifische Lectine, Rezeptoren oder ähnliche Moleküle.

Die folgenden Beispiele erläutern die Erfindung.

### Beispiel 1 a)

### Herstellung von anti-F1+2 Latexreagenz

Die Herstellung von Latexreagenzien erfolgte nach Kapmeyer W.H. et al., J. Clin. Lab. Anal. 2: 76-83 (1988). 1 ml eines Pfropfpolymerisats wurde mit 0,1 ml Antikörperlösung (spezifische Antikörper vom Kaninchen gegen den C-Terminus des Prothrombinfragments F1+2 (zur Herstellung siehe EP 0 303 983; Konzentration: 0,5 mg/ml) und 0,05 ml einer 20 %-igen wäßrigen Tween® 20 Lösung gemischt. Zur Aktivierung der geschützten Aldehydgruppen auf dem Schalenpolymerisat wurde die Suspension mit ca. 0,01 ml einer 1 N HCI-Lösung auf einen pH von 2,5 eingestellt. Nach einer 30minütigen Inkubation bei Raumtemperatur wurden 25 mg Natriumborhydrid in 1 ml einer 1 M Natriumhydrogenphosphat-Lösung (pH 6,5) gelöst und davon 0,25 ml zur Beschichtungslösung zugegeben. Die Kopplung des Antikörpers an die aktivierten Aldehydgruppen erfolgte über 1 Stunde bei Raumtemperatur. Anschließend wurde das Latex-Antikörper Konjugat zentrifugiert (Beckman Zentrifuge, 40000xg, 30 Minuten) und das Pellet in 1,5 ml eines 0,1 molaren Glycin-Puffers (pH 8,2; enthaltend 0,17 M NaCI und 0,5% Tween® 20) resuspendiert. Die Lösung wurde mit Ultraschall für ca. 5 Sekunden behandelt (Bronson Sonifier B 15). Diese Stammlösung wurde bei +4°C gelagert.

### Beispiel 1 b)

### Herstellung von F1+2-Peptid Konjugaten mit alkalischer Phosphatase (AP) oder Meerrettich-Peroxidase (POD)

### a) Prinzip des Verfahrens

Die Herstellung von Enzym-/Peptid-Konjugaten erfolgt nach gängigen Prinzipien mittels hetero-bifunktionaler Linker, wie etwa beschrieben in P. Tijssen (Laboratory techniques in biochemistry and molecular biology; Vol. 15, Elsevier Science Publishers B.V., Amsterdam - New York - Oxford, 1988). Dem synthetisch hergestellten Antigen (F1+2 Peptid; Fa. Behringwerke AG) wurde am Aminoterminus ein Cystein angefügt (siehe EP 0 303 983). Die Verbindung zwischen der SH-Gruppe des N-terminalen Cysteins des Peptids und den Aminofunktionen der N-Termini der zu konjugierenden Enzyme erfolgt mittels m-Maleimidbuturyl-N-hydroxysuccinimid-Ester (MBS; Fa. Serva). Zur Vermeidung der Bildung von Enzym/ Enzym-Konjugaten werden etwaige freie SH-Gruppen der Enzyme vor der eigentlichen Kopplung mit N-Ethylmaleimid (NEM; Fa. Serva) geschützt.

### b) Herstellung von SH-geschützten Enzymen

8,8 mg Meerrettich-Peroxidase (POD) bzw. 15 mg alkalische Phosphatase (AP) (beides von Fa. Boehringer Mannheim) werden in 1 ml Kopplungspuffer (0,1 mol/l Natriumphosphat Puffer, 5 mM EDTA, pH 6,0; bei AP zusätzlich 1 mmol/l MgCl₂ und 1 µmol/l ZnCl₂) gelöst. Bei Raumtemperatur werden unter Rühren tropfenweise 0,1 ml NEM-Lösung (18 mg/ml in N,N-Dimethylformamid) zugegeben. Das Gefäß wird verschlossen 1 Stunde unter Rühren bei Raumtemperatur inkubiert. Anschließend wird die Lösung gegen Reaktionspuffer (0,1 mol/l Natriumphosphat Puffer, pH 8,0; bei AP zusätzlich 1 mmol/l MgCl₂ und 1 µmol/l ZnCl₂) dialysiert und, falls notwendig, in Centrikons (Fa. Amicon; Ausschlußgröße <10 kD) auf ca. 1 ml ankonzentriert.

### c) Einfügen einer reaktiven Maleimidfunktion in die Enzyme

Die SH-geschützten Enzyme in ca. 1 ml Reaktionspuffer aus Beispiel 1.b) werden mit 0,1 ml MBS-Lösung (100 mg/ml *m*-Maleimidbuturyl-*N*-hydroxysuccinimid-Ester in *N,N*-Dimethylformamid) tropfenweise unter Rühren bei Raumtemperatur versetzt. Es wird 1 Stunde lang nachgerührt und die Lösung gegen Kopplungspuffer (0,1 mol/l Natriumphosphat Puffer, 5 mM EDTA, pH 6,0; bei AP zusätzlich 1 mmol/l MgCl₂ und 1 µmol/l ZnCl₂) dialysiert. Falls notwendig werden die aktivierten Enzymlösungen auf ca. 2 ml mittels Centrikons (Fa. Amicon) ankonzentriert.

### d) Kopplung des Peptids an die aktivierten Enzyme

2 mg des F1+2-Peptids werden in 2 ml Kopplungspuffer (0,1 mol/l Natriumphosphat Puffer, 5 mM EDTA, pH 6,0; bei AP zusätzlich 1 mmol/l MgCl₂ und 1 µmol/l ZnCl₂) gelöst. Anschließend werden 2 ml der aktivierten Enzymlösung aus Beispiel 1b.c) unter Rühren zugegeben. Das verschlossene Gefäß wird für 1 Stunde bei Raumtemperatur unter Rühren inkubiert.

### e) Sättigung der verbliebenen Maleimid-Gruppen

Die Kopplungslösung aus Beispiel 1b.d) wird mit 400 µl frisch angesetzter Cysteinlösung (10 mmol/l in 0,1 mol/l Natriumphosphat Puffer, 5 mM EDTA, pH 6,0; bei AP zusätzlich 1 mmol/l MgCl₂ und 1 µmol/l ZnCl₂) versetzt und für ca. 10 min nachgerührt.

### f) Dialyse und Aufbewahrung der F1+2-Peptid/Enzym-Konjugate

Die Kopplungslösung aus Beispiel 1b.e) wird gegen Konjugat-Dialysemedium (5 mmol/l Tris/HCl, 0,9 g/l Phenol, pH 7,4; bei AP zusätzlich 1 mmol/l MgCl₂ und 1 µmol/l ZnCl₂) dialysiert und bei -20°C aufbewahrt.

### Beispiel 1 c)

### Direkte Bestimmung von F1+2-Peptid mittels F1+2 Peptid/POD-Konjugat

50 µl eines nach Beispiel 1 hergestellten anti-F1+2-Latexreagenzes in einer Verdünnung der Stammlösung von 1:30 in Testmedium (0,02 mol/l Tris/HCl, 9 g/l NaCI, 0,5 g/l Tween 20, pH 8,2) werden mit 25 µl F1+2 Peptid (Probe) versetzt und 15 Minuten bei +37 °C inkubiert. Anschließend werden 25 µl eines F1+2-Peptid/POD-Konjugats (hergestellt gemäß Beispiel 1b mit Meerrettich-Peroxidase; 1:5000 verdünnt in Testmedium) zugemischt und weitere 10 Minuten bei +37 °C inkubiert. Eine Lösung von anti-Kaninchen Antikörpern von der Ziege (Fa. Behringwerke; 0,056 g/l in 10 mmol/l Tris/HCl, 0,45 g/l NaCl, 0,25 g/l Tween 20, 50 g/l Polyethylenglycol 6000, pH 8,0) wird zugegeben und nochmals 3 Minuten bei +37 °C inkubiert. Anschließend werden die gefällten Partikel durch Zentrifugation für 3 Minuten bei 400 x g abgetrennt. Aus dem Überstand werden 10 µl entnommen und mit 100 µl Substratlösung für POD (Fa. Behringwerke AG) lichtgeschützt bei Raumtemperatur für 30 Minuten inkubiert. Die Substratreaktion wird durch Zugabe von 100 µl 0,5 N Schwefelsäure gestoppt und die Extinktion bei 492 nm gemessen. Die erhaltenen Extinktionen in einem Meßbereich von 1,9 bis 19190 nmol/l F1+2 Peptid sind in Tabelle 1 aufgeführt (siehe auch Beispiel 2).

Dieser Versuchsaufbau wurde im Labor in Eppendorf Reaktionsgefäßen durchgeführt. Bei entsprechender Programmierung von klinisch-chemischen Zentrifugalanalysatoren ist dieser Aufbau auch an diesen Geräten durchführbar und bietet somit die Möglichkeit der Automatisierung.

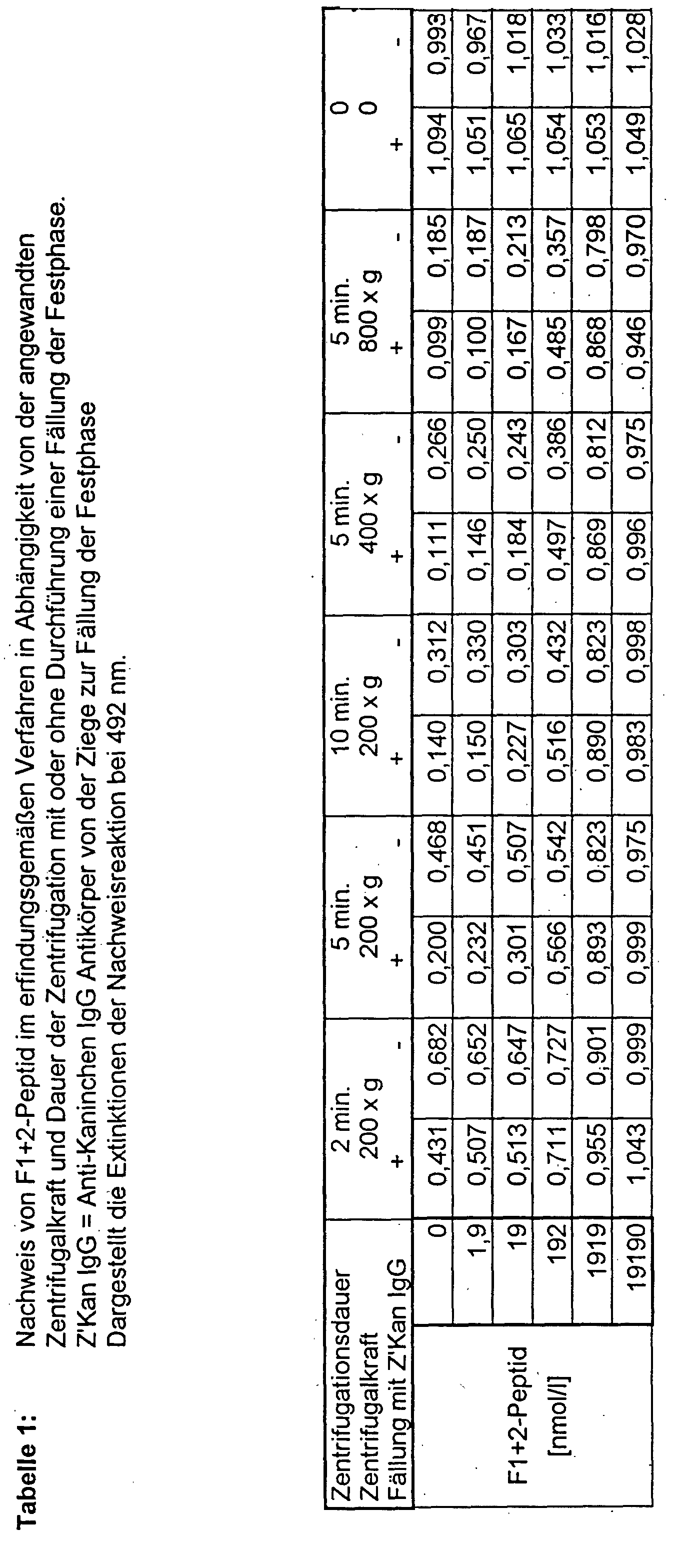

### Beispiel 2

### Erhöhung der analytischen Sensitivität durch die erfindungsgemäße Fällung der Festphase

Gemäß dem Testaufbau in Beispiel 1 c) wurden Referenzkurven mit F1+2 Peptid im Konzentrationsbereich von 1,9 bis 19190 nmol/l aufgenommen. In Variation zum Testaufbau in Beispiel 3 wurden auch Bestimmungen ohne Fällung des anti-F1+2-Latexreagenzes mittels anti-Kaninchenantikörpern durchgeführt. Des weiteren wurden die Zentrifugationsbedingungen hinsichtlich der Beschleunigung (200 bis 400 x g) und der Dauer (2 bis 10 Minuten) variiert. Die Ergebnisse sind in Tabelle 1 zusammengefaßt.

Insbesondere bei niedrigen Zentrifugalbeschleunigungen, wie sie typischerweise an klinisch-chemischen Zentrifugalanalysatoren erreicht werden, kann eine Bestimmung von F1+2 nur mittels der erfindungsgemäßen Fällung der Festphase erzielt werden (Fig. 1). Unter Zusatz von Fällungsantikörpern verlaufen die Referenzkurven steiler, d.h. Präzision als auch Sensitivität sind besser. Zudem ist der Hintergrund (ohne F1+2 Peptid) niedriger, d.h. das Signal zu Hintergrundverhältnis verbessert. Eine geringe Sedimentation ohne Zusatz von Fällungsagenzien ist auch sinnvoll, da die Festphase während der Inkubations- und Mischungsphasen nicht sedimentieren soll um möglichst kurze Diffusionswege von Analyt und Analyt/Enzym-Konjugat zu erhalten. Aber auch bei höheren Zentrifugalbeschleunigungen (Fig. 2 und 3) bleibt diese Differenz bestehen. Da die Sedimentation durch das Produkt aus Beschleunigung und Zeit bestimmt wird, läßt sich die analytische Sensitivität sowohl durch länger andauernde Beschleunigungen (Fig. 4; bei 200 x g für 2 bis 10 Minuten in Anwesenheit von Fällungsantikörpern), als auch durch höhere Beschleunigungen (Fig. 5, für 5 Minuten bei 200 bis 800 x g in Anwesenheit von Fällungsantikörpern) erreichen.

### Beispiel 3

### Einstellung des Meßbereichs durch Variation der Konzentration des Latexreagenzes

Die Erhöhung der analytischen Sensitivität und Präzision durch wie unter Beispiel 2 beschriebenen Erhöhung der Zentrifugalbeschleunigung und/oder der Dauer der Zentrifugation sind in der Regel durch den Geräteaufbau eingeschränkt. Außerdem wird der Testdurchsatz bei zu langen Zentrifugationszeiten deutlich vermindert. Wesentlich günstiger ist es durch die Verringerung des Latexreagenzes und damit verbunden auch des Analyt/Enzym-Konjugats den Meßbereich zu verändern.

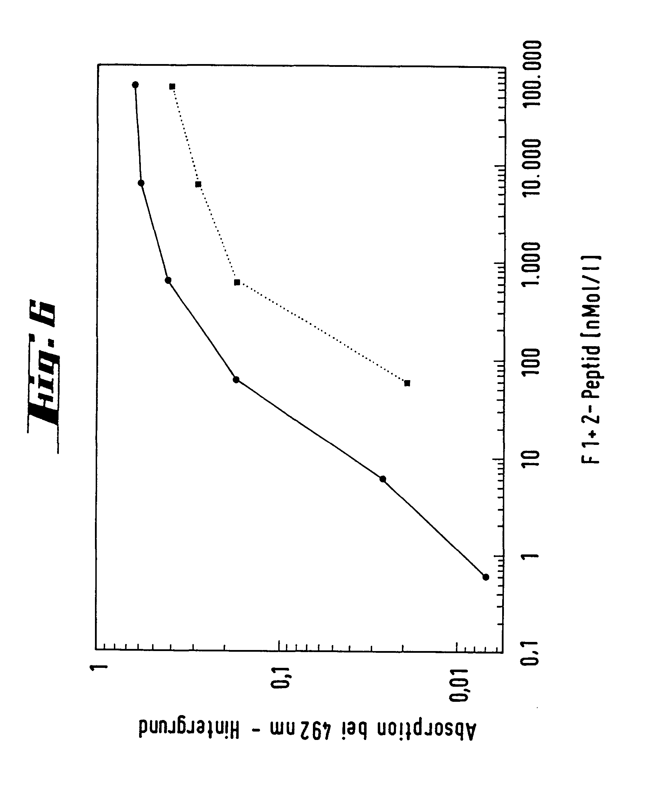

Nach dem erfindungsgemäßen Verfahren wurde mit F1+2-Peptid eine Referenzkurve im Bereich von 0,6 bis 57575 nmol/l erstellt. In Variation zum Testaufbau nach Beispiel 1 c) erfolgte die Durchführung mit 1:15 bzw. 1:30 verdünntem anti-F1+2-Latexreagenz und 1:3000 bzw. 1:5000 verdünntem F1+2-Peptid/POD-Konjugat. Zudem wurde die Zentrifugation bei 3000 x g durchgeführt. Die Substratinkubation hingegen auf nur 15 Minuten beschränkt. Durch die höhere Verdünnung der reaktiven Reagenzien (Festphase und Konjugat) wurde das Meßfenster und somit die analytische Sensitivität von ca. 50 - 50000 nmol/l auf ca. 2 bis 2000 nmol/l verschoben (Tabelle 2). Dies ist in Figur 6 verdeutlicht, wobei bei der grafischen Darstellung auf Grund der unterschiedlichen Konjugatkonzentration das Hintergrundsignal (0 ng/ml) berücksichtigt wurde.

**Tabelle 2:**

| Nachweis von F1+2 Peptid im erfindungsgemäßen Verfahren in Abhängigkeit von der Konzentration an Latexreagenz (1:30 bzw. 1:15 verdünnt) und der korrespondierenden Konjugatkonzentration (1:5000 bzw. 1:3000 verdünnt). | | | |
|---|---|---|---|
| Dargestellt die Extinktionen der Nachweisreaktion bei 492 nm. | | | |
| Latexreagenz-Verdünnung | | 1:30 1:5000 | 1:15 1:3000 |
| Konjugat-Verdünnung | | | |
| F1+2-Peptid [nmol/l] | 0 | 0,190 | 0,110 |
| | 0,6 | 0,197 | n.d. |
| | 5,8 | 0,216 | n.d. |
| | 58 | 0,365 | 0,129 |
| | 576 | 0,612 | 0,283 |
| | 5757 | 0,778 | 0,393 |
| | 57576 | 0,819 | 0,405 |

### Beispiel 4

### Bestimmung von F1+2-Peptid unter Verwendung einer nachgeschalteten Verstärkerreaktion

Zur Erhöhung der analytischen Sensitivität wurde die im Überstand verbliebenen Enzymaktivität nicht direkt, sondern durch Nachschaltung einer Verstärkerreaktion bestimmt. Zur Verstärkung wurde das System nach Harborn, St. et al. (Anal. Biochem. 206: 119-124 (1992); PCT WO 90/01559) verwendet. Das System beruht auf der Spaltung des phosphorylierten Flavin-Adenin-dinucleotids, das nur in der dephosphorylierten Form als Coenzym für eine Aminosäureoxidase dienen kann. Die Aktivität der Aminosäureoxidase wird nachgewiesen durch das bei der Oxidation der Aminosäure freigesetzte Wasserstoffperoxid, das seinerseits als Substrat für eine zugegebene Peroxidase dient, die das Substrat (4-Aminoantipyrin und 3,5-Dichloro-2-hydroxy-benzen-sulphonat) umsetzt, das letztlich eine bei 540 nm meßbare Farbreaktion ergibt. Diese Farbreaktion ist somit direkt abhängig von der Menge an gebildetem Coenzym, das die Aktivität der Apo-Aminosäureoxidase erheblich verstärkt. Die Freisetzung des Coenzyms geschieht durch Phosphatasen, weshalb in diesem Fall gemäß Beispiel 2 ein F1+2-Peptid/AP-Konjugat hergestellt und nach dem erfindungsgemäßen Verfahren eingesetzt wurde. Die übrigen zur Nachweisreaktion benötigten Reagenzien wurden gebrauchsfertig von der Fa. London Biotechnology, London, bezogen.

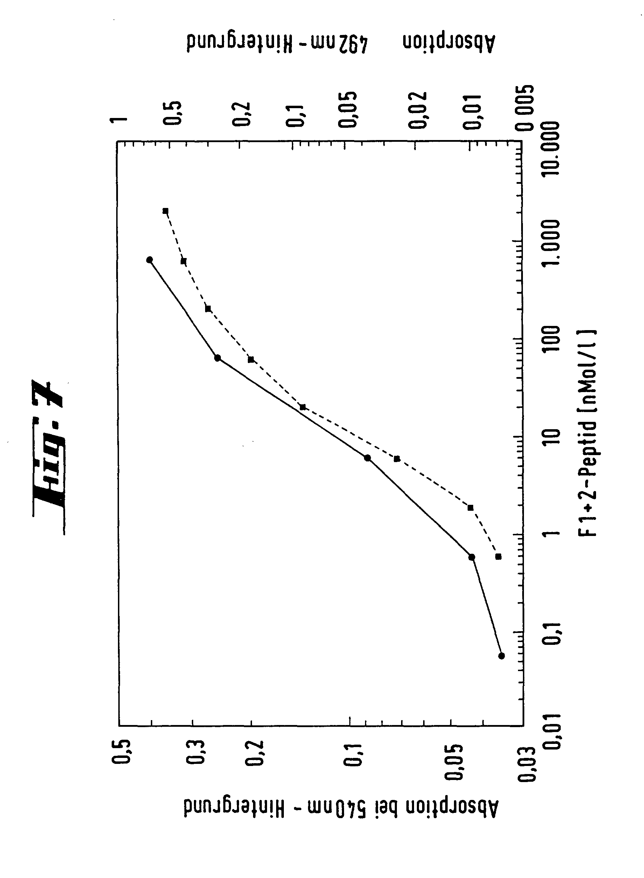

Die Testdurchführung erfolgte gemäß Beispiel 1 c), allerdings wurde gegenüber Beispiel 3 die Konzentration an anti-F1+2-Latexreagenz und entsprechend F1+2-Peptid-AP Konjugat mit Verdünnungen der Ausgangslösungen auf 1:500 bzw. 1:10000 noch weiter reduziert. In Tabelle 3 sind zum Vergleich die Ergebnisse des Nachweises von F1+2-Peptid mit und ohne (aus Beispiel 3) Verstärkersystem aufgeführt. Ein Vergleich der Ergebnisse unter Berücksichtigung des jeweiligen Test-spezifischen Hintergrundes (0-Wert) ist in Figur 7 dargestellt. Der Einsatz einer zusätzlichen Kette von Reaktionen zum Nachweis des im Überstand verbliebenen Konjugats führt zu einer Verbesserung der Nachweisempfindlichkeit in diesem Beispiel von ca. 2 nmol/l auf mindestens 0,2 nmol/l.

**Tabelle 3:**

| Nachweis von F1+2- Peptid im erfindungsgemäßen Verfahren ohne und mit einem nachgeschalteten Verstärkersystem. Ohne Verstärkersystem wurde ein F1+2/POD-, mit Verstärkersystem ein F1+2/AP-Konjugat eingesetzt . (POD = Meerrettich-Peroxidase; AP = Alkalische Phospatase) | | | |
|---|---|---|---|
| Dargestellt sind die mit dem jeweiligen Nachweissystem erhaltenen Extinktionen bei 492 (POD) bzw. 540 (AP) nm | | | |
| | Konjugat Verstärker system | F1+2/POD ohne | F1+2/AP mit |
| F1+2-Peptid [nmol/l] | 0 | 0,190 | 0,618 |
| | 0,0058 | n.d. | 0,621 |
| | 0,058 | n.d. | 0,653 |
| | 0,58 | 0,197 | 0,661 |
| | 1,9 | 0,200 | n.d. |
| | 5,8 | 0,216 | 0,706 |
| | 19 | 0,279 | n.d. |
| | 58 | 0,365 | 0,868 |
| | 192 | 0,498 | n.d. |
| | 576 | 0,612 | 1,018 |
| | 1919 | 0,721 | n.d. |
| | 5757 | 0,778 | 1,131 |

Die Figuren zeigen:

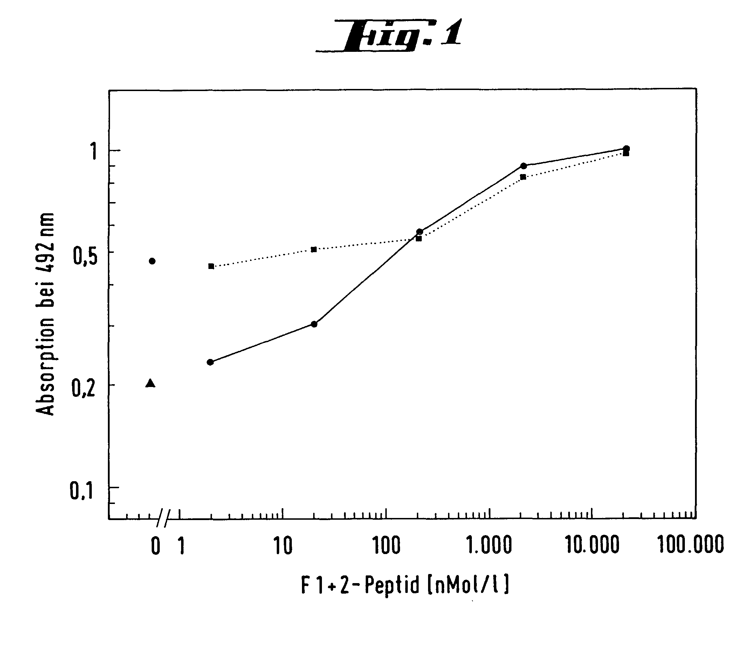

- Figur 1:: Effekt der Präzipitation der Festphase durch bindungsfähige Liganden im erfindungsgemäßen Verfahren bei einer Zentrifugalkraft von 200 x g und 5minütiger Zentrifugation.
Dargestellt sind die in der Nachweisreaktion erhaltenen Extinktionen bei 492 nm in Abhängigkeit der F1+2-Peptid Konzentration im Ansatz bei Anwendung der erfindungsgemäßen Präzipitation der Festphase (durchgezogene Linie) bzw. ohne Präzipitation (gestrichelte Linie).

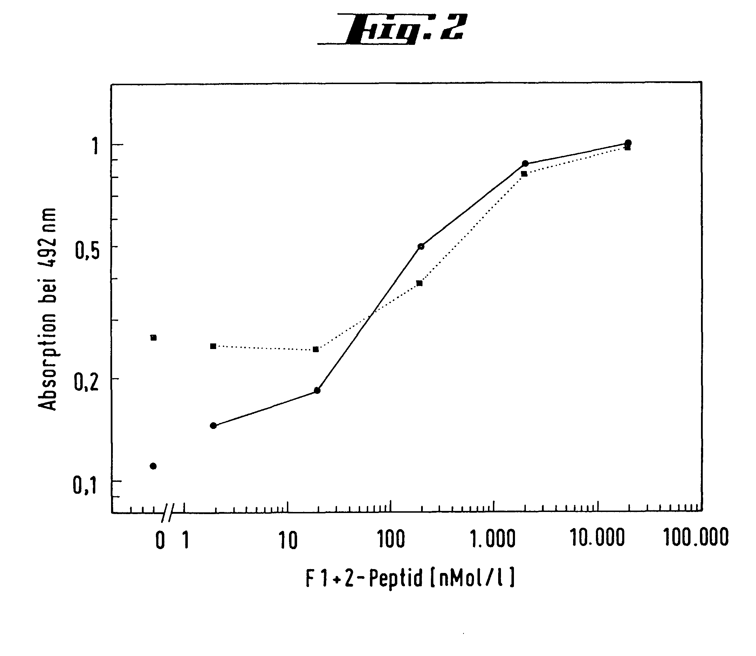

- Figur 2:: Effekt der Präzipitation der Festphase durch bindungsfähige Liganden im erfindungsgemäßen Verfahren bei einer Zentrifugalkraft von 400 x g und 5minütiger Zentrifugation.
Dargestellt sind die in der Nachweisreaktion erhaltenen Extinktionen bei 492 nm in Abhängigkeit der F1+2-Peptid Konzentration im Ansatz bei Anwendung der erfindungsgemäßen Präzipitation der Festphase (durchgezogene Linie) bzw. ohne Präzipitation (gestrichelte Linie).

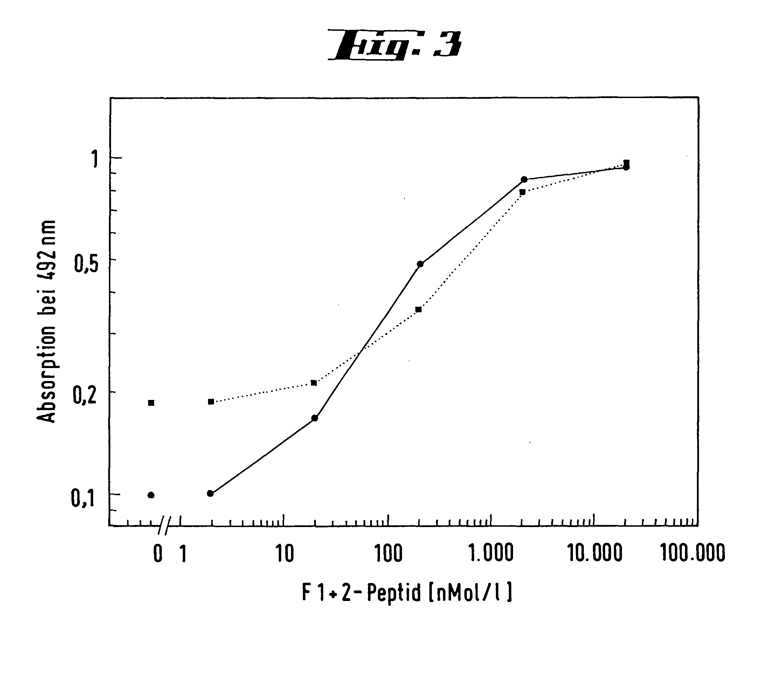

- Figur 3:: Effekt der Präzipitation der Festphase durch bindungsfähige Liganden im erfindungsgemäßen Verfahren bei einer Zentrifugalkraft von 800 x g und 5minütiger Zentrifugation.
Dargestellt sind die in der Nachweisreaktion erhaltenen Extinktionen bei 492 nm in Abhängigkeit der F1+2-Peptid Konzentration im Ansatz bei Anwendung der erfindungsgemäßen Präzipitation der Festphase (durchgezogene Linie) bzw. ohne Präzipitation (gestrichelte Linie).

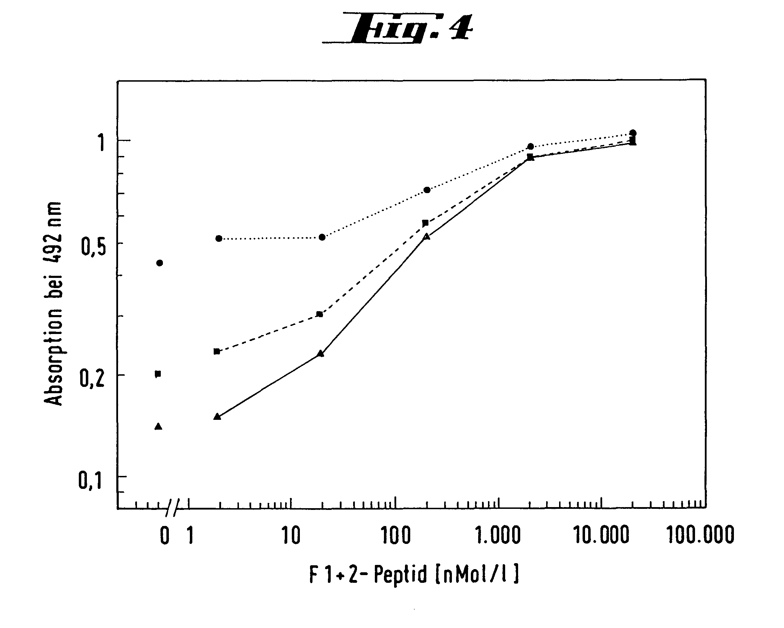

- Figur 4:: Abhängigkeit der Nachweisreaktion mit F1+2 Peptid im erfindungsgemäßen Verfahren von.der Dauer der Zentrifugation bei einer Zentrifugalkraft von 200 x g.
Dargestellt sind die in der Nachweisreaktion erhaltenen Extinktionen bei 492 nm in Abhängigkeit der F1+2-Peptid Konzentration im Ansatz bei 2-(gepunktete Linie), 5- (gestrichelte Linie) bzw. 10-minütiger Zentrifugation (durchgezogene Linie) der präzipitierten Festphase.

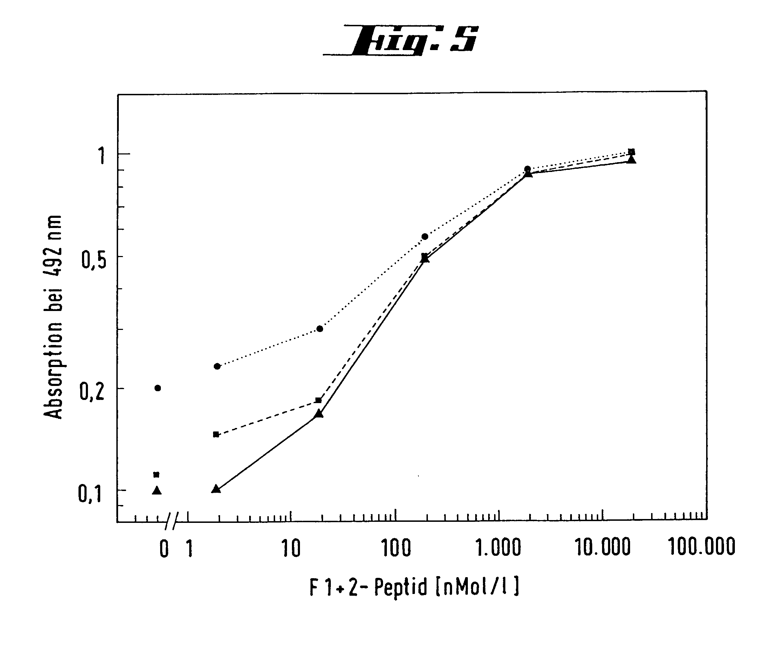

- Figur 5:: Abhängigkeit der Nachweisreaktion mit F1+2 Peptid im erfindungsgemäßen Verfahren von der Zentrifugalkraft 5minütiger Zentrifugation. Dargestellt sind die in der Nachweisreaktion erhaltenen Extinktionen bei 492 nm in Abhängigkeit der F1+2-Peptid Konzentration im Ansatz bei 200 x g (gepunktete Linie), 400 x g (gestrichelte Linie) bzw. 800 x g (durchgezogene Linie).
- Figur 6:: Abhängigkeit des Meßfensters und der Nachweisempfindlichkeit für F1+2 Peptid im erfindungsgemäßen Verfahren durch Variation der Konzentration der reaktiven Komponenten.
Dargestellt sind die in der Nachweisreaktion erhaltenen Extinktionen bei 492 nm in Abhängigkeit der F1+2-Peptid Konzentration im Ansatz bei einer Konzentration von anti-F1+2-Latexreagenz und F1+2-Peptid/POD-Konjugat von 1:30 und 1:5000 (verdünnter Ansatz; durchgezogene Linie) bzw. von 1:15 und 1:3000 (konzentrierter Ansatz; gestrichelte Linie) nach Abzug der testspezifischen Hintergrundreaktion (0 ng/ml).
- Figur 7:: Nachweis von F1+2 Peptid im erfindungsgemäßen Verfahren mit und ohne nachgeschaltetes Reaktionssystem zur Verstärkung der Nachweisempfindlichkeit.
Dargestellt sind die Extinktionen bei 492 nm beim unverstärkten Nachweis mittels F1+2-Peptid/Meerrettich-Peroxidase-Konjugat (gestrichelte Linie) bzw. bei 540 nm beim verstärkten Nachweissystem mittels F1+2-Peptid/Alkalische Phosphatase-Konjugat (durchgezogene Linie) nach Abzug des testspezifischen Hintergrundsignals (ohne F1+2-Peptid im Ansatz).

## Patentansprüche

1. Verfahren zum immunchemischen Nachweis eines Analyten in einer Probe einer biologischen Flüssigkeit, das folgende Schritte einschließt
a) Immobilisieren eines ersten unmarkierten spezifischen Bindungspartners für den Analyten an eine pipettierbare partikuläre Festphase,
b) Zugeben der Probe zu dem immobilisierten unmarkierten spezifischen Bindungspartner,
c) erste Inkubation des Reaktionsansatzes,
d) Zugeben einer definierten Menge einer markierten spezifischen Nachweissubstanz,
e) zweite Inkubation des Reaktionsansatzes,
f) Präzipitation der Festphase durch Zugabe von mindestens einer Substanz aus der Gruppe, die folgende Substanzen umfaßt:
i) ein gegen die Festphase gerichteter spezifischer Bindungspartner,
ii) ein gegen die nachzuweisende Substanz gerichteter spezifischer Bindungspartner,
iii) ein gegen eine auf der Festphase immobilisierte Ankersubstanz gerichteter spezifischer Bindungspartner,
wobei die spezifischen Bindungspartner bevorzugterweise von einer anderen Spezies sind als der erste spezifische Bindungspartner.
g) Zentrifugation des Reaktionsansatzes bei bevorzugterweise 10 bis 1000 x g, besonders bevorzugterweise bei 200 bis 800 x g,
h) Überführung zumindest eines Teils des in Schritt g) entstandenen Überstandes in eine zweite Meßkammer,
i) Starten einer Nachweisreaktion in der zweiten Meßkammer,
j) Bestimmung der Analytkonzentration aus der Nachweisreaktion.

2. Verfahren nach Anspruch 1, wobei die markierte spezifische Nachweissubstanz ein markierter Analyt ist.

3. Verfahren nach Anspruch 1, wobei die markierte spezifische Nachweissubstanz mindestens ein weiterer markierter spezifischer Bindungspartner für den Analyten ist.

4. Verfahren nach mindestens einem der Ansprüche 1-3, wobei es sich bei dem nachzuweisenden Analyten um Haptene oder um Proteine mit einem oder mehreren spezifischen Epitopen handelt.

5. Verfahren nach Anspruch 4 wobei der Analyt ein Protein mit nur einem spezifischen Epitop ist.

6. Verfahren nach Anspruch 2, wobei der Analyt aus natürlichen, z. B. tierischen oder humanem Material isoliert wurde, bzw. gentechnisch oder synthetisch hergestellt wurde.

7. Verfahren nach Anspruch 2, wobei der Analyt nur zum Teil dem gesuchten Analyt entspricht oder chemisch oder biochemisch modifiziert wurde.

8. Verfahren nach Anspruch 3, wobei der affine Bindungspartner ausgewählt ist aus der Gruppe die folgende Substanzen umfaßt: Antikörper, Lectin, Avidin, Streptavidin, Biotin oder Derivate davon, Komplementfaktor C1, Mannanbindendes Protein, ein Cofaktor.

9. Verfahren nach Anspruch 1, wobei es bei der nachweisbaren Markierung um ein radiochemisch nachweisbares Element, eine fluoreszierende oder chemilumineszente Verbindung, um ein Enzym oder um einen Cofaktor handelt, die in einer geeigneten Folgereaktion nachgewiesen werden können.

10. Verfahren nach Anspruch 9, wobei als Markierung ein Enzym, bevorzugterweise alkalische Phosphatase oder Meerrettich Peroxidase verwendet wird.

11. Verfahren nach Anspruch 1, wobei die Bildung von Immunkomplexen durch die Zugabe von Dextransulfat und/oder Polyethylenglycol beschleunigt wird.

12. Verfahren nach Anspruch 2, wobei die Präzipitationssubstanz gegen den Analyten gerichtet ist.

13. Verfahren nach Anspruch 12, wobei das Konjugat aus einem markierten modifizierten Analyten besteht, der nicht mit der Präzipitationssubstanz reagiert.

14. Verfahren nach Anspruch 1, wobei die Präzipitationssubstanz gegen eine zusätzlich an den ersten unmarkierten spezifischen Bindungspartner gekoppelte Substanz gerichtet ist.

15. Verfahren nach einem der Ansprüche 1 bis 14, wobei die Präzipitationssubstanz zur Verstärkung der Sedimentationsgeschwindigkeit selbst an eine unlösliche Festphase immobilisiert ist.

16. Verfahren nach Anspruch 15, wobei der erste unmarkierte spezifische Bindungspartner nicht an eine Festphase gebunden ist.

17. Verfahren nach Anspruch 15, wobei die zur Immobilisierung verwendet Festphase ausgewählt ist aus der Gruppe der dem Fachmann an sich bekannten Partikel wie: Glas, Gelatine, Agarose, Lipide, Erythrozyten, Blutplättchen, Leukozyten, Metall Kolloiden, synthetische Materialien, die bevorzugterweise magnetisierbar sind.

18. Verfahren nach Anspruch 17, wobei die synthetischen Partikel ausgewählt sind aus der Gruppe bestehend aus Polystyrol, Polydextran, Polypropylen, Polyvinylchlorid, Poyvinylidienfluorid, Poylacrylamid oder Copolymeren aus Styrol-Butadien, Styrol-Methacrylsäure oder Methacrylat-Methacrylsäure.

19. Verfahren nach Anspruch 1, wobei die Präzipitationssubstanz ein Antikörper, Lectin, Substrat oder dessen Analoga, Avidin, Streptavidin, Biotin oder Derivate davon, Komplementfaktor C1, Mannan-bindendes Protein, ein Cofaktor oder eine andere Substanz ist, die mit dem gewünschten Reaktionspartner ein spezifische Verbindung eingeht.

20. Verfahren nach Anspruch 19, wobei die Präzipitationssubstanz ein Antikörper ist.

21. Verfahren nach Anspruch 1, wobei die Präzipitationsreaktion (f) durch Änderung des Reaktionsmilieus beschleunigt wird.

22. Verfahren nach Anspruch 21, wobei die Beschleunigung der Trennung durch eine Änderung des pH-Wertes erreicht wird.

23. Verfahren nach Anspruch 1, wobei Zellen als pipettierbare partikuläre Festphase eingesetzt werden und als Präzipitationssubstanzen Antikörper gegen ein oder mehrere Oberflächenantigene dieser Zellen.

24. Verfahren nach Anspruch 1, wobei magnetisierbare Partikel als pipettierbare partikuläre Festphase eingesetzt werden und als Präzipitationssubstanzen magnetische Partikel bzw. im umgekehrten Verfahren magnetische Partikel als Festphase und andere magnetische oder magnetisierbare Partikel zur Präzipitation verwendet werden.

## Claims

1. A method for the immunochemical detection of an analyte in a sample of a biological fluid, which method includes the following steps
a) immobilizing a first, unlabeled specific binding partner for the analyte on a pipettable, particulate solid phase,
b) adding the sample to the immobilized, unlabeled specific binding partner,
c) carrying out the first incubation of the reaction mixture,
d) adding a defined quantity of a labeled, specific detection substance,
e) carrying out the second incubation of the reaction mixture,
f) precipitating the solid phase by adding at least one substance from the group which comprises the following substances:
i) a specific binding partner which is directed against the solid phase,
ii) a specific binding partner which is directed against the substance to be detected,
iii) a specific binding partner which is directed against an anchoring substance which is immobilized on the solid phase,
where the specific binding partners are preferably of a species which is different from that of the first specific binding partner,
g) centrifuging the reaction mixture, preferably at from 10 to 1000 × g, and particularly preferably at from 200 to 800 × g,
h) transferring at least some of the supernatant arising in step g) to a second measuring chamber,
i) starting a detection reaction in the second measuring chamber,
j) determining the analyte concentration from the detection reaction.

2. The method as claimed in claim 1, wherein the labeled, specific detection substance is a labeled analyte.

3. The method as claimed in claim 1, wherein the labeled, specific detection substance is at least one additional, labeled specific binding partner for the analyte.

4. The method as claimed in at least one of claims 1-3, wherein the analyte to be detected is a hapten or a protein having one or more specific epitopes.

5. The method as claimed in claim 4, wherein the analyte is a protein having only one specific epitope.

6. The method as claimed in claim 2, wherein the analyte has either been isolated from natural, for example animal or human, material, or else has been prepared by genetic manipulation or synthetically.

7. The method as claimed in claim 2, wherein the analyte only partially corresponds to the soughtafter analyte, or has been chemically or bio-chemically modified.

8. The method as claimed in claim 3, wherein the high-affinity binding partner is selected from the group comprising the following substances: antibody, lectin, avidin, streptavidin, biotin, or derivatives thereof, complement factor C1, mannan-binding protein or a cofactor.

9. The method as claimed in claim 1, wherein the detectable label is a radiochemically detectable element, a fluorescent or chemiluminescent compound, or an enzyme or a cofactor which can be detected in an appropriate subsequent reaction.

10. The method as claimed in claim 9, wherein an enzyme, preferably alkaline phosphatase or horseradish peroxidase, is used as the label.

11. The method as claimed in claim 1, wherein the formation of immune complexes is accelerated by adding dextran sulfate and/or polyethylene glycol.

12. The method as claimed in claim 2, wherein the precipitation substance is directed against the analyte.

13. The method as claimed in claim 12, wherein the conjugate comprises a labeled modified analyte which does not react with the precipitation substance.

14. The method as claimed in claim 1, wherein the precipitation substance is directed against a substance which is additionally coupled to the first, unlabeled specific binding partner.

15. The method as claimed in one of claims 1 to 14, wherein the precipitation substance is itself immobilized on an insoluble solid phase in order to increase the speed of sedimentation.

16. The method as claimed in claim 15, wherein the first, unlabeled specific binding partner is not bound to a solid phase.

17. The method as claimed in claim 15, wherein the solid phase used for the immobilization is selected from the group of particles known per se to the person skilled in the art, such as: glass, gelatine, agarose, lipids, erythrocytes, blood platelets, leucocytes, metal colloids and synthetic materials which are preferably magnetizable.

18. The method as claimed in claim 17, wherein the synthetic particles are selected from the group consisting of polystyrene, polydextran, polypropylene, polyvinyl chloride, polyvinylidene fluoride, polyacrylamide or with styrene-butadiene, styrene-methacrylic acid or methacrylate-methacrylic acid copolymers.

19. The method as claimed in claim 1, wherein the precipitation substance is an antibody, lectin, substrate or its analogs, avidin, streptavidin, biotin or derivatives thereof, complement factor C1, mannan-binding protein, a cofactor or another substance which enters into a specific bond with the desired reaction partner.

20. The method as claimed in claim 19, wherein the precipitation substance is an antibody.

21. The method as claimed in claim 1, wherein the precipitation reaction (f) is accelerated by altering the reaction medium.

22. The method as claimed in claim 21, wherein the separation is accelerated by altering the pH.

23. The method as claimed in claim 1, wherein cells are employed as the pipettable, particulate solid phase, and antibodies against one or more surface antigens of these cells are employed as the precipitation substances.

24. The method as claimed in claim 1, wherein magnetizable particles are employed as the pipettable, particulate solid phase, and magnetic particles are employed as the precipitation substances, or, in the reverse method, magnetic particles are used as the solid phase, and other magnetic or magnetizable particles are used for the precipitation.

## Revendications

1. Procédé pour la détection immunochimique d'un analyte dans un échantillon d'un liquide biologique, comprenant les étapes suivantes :
a) immobilisation d'un premier partenaire de liaison spécifique non marqué pour l'analyte, sur une phase solide particulaire apte au pipetage,
b) addition de l'échantillon au partenaire de liaison spécifique non marqué immobilisé,
c) première incubation du mélange réactionnel,
d) addition d'une quantité définie dune substance de détection spécifique marquée,
e) seconde incubation du mélange réactionnel,
f) précipitation de la phase solide par addition d'au moins une substance choisie dans le groupe comprenant les substances suivantes :
I) un partenaire de liaison spécifique dirigé contre la phase solide,
II) un partenaire de liaison spécifique dirigé contre la substance à détecter,
III) un partenaire de liaison spécifique dirigé contre une substance d'ancrage immobillsée sur la phase solide,
les partenaires de liaison spécifique étant de préférence d'une aspéce différente de celle du premier partenaire de liaison spécifique,
g) centrifugation du mélange réactionnel, de préférence de 10 à 1 000 g, de façon particulièrement préférée de 200 à 800 g,
h) transfert d'au moins une partie du surnageant, formé dans l'étape g), dans une seconde chambre de mesure,
i) déclenchement d'une réaction de détection dans la seconde chambre de mesure,
j) détermination de la concentration d'analyte à partir de la réaction de détection.

2. Procédé selon la revendication 1, dans lequel la substance de détection spécifique marquée est un analyte marqué.

3. Procédé selon la revendication 1, dans lequel la substance de détection spécifique marquée est au moins un autre partenaire de liaison marqué spécifique pour l'analyte.

4. Procédé selon au moins l'une des revendications 1 à 3, dans lequel, en ce qui concerne l'analyte à détecter, il s'agit d'haptènes ou de protéines comportant un ou plusieurs épitopes spécifiques.

5. Procédé selon la revendication 4, dans lequel l'analyte est une protéine ne comportant qu'un seul épitope spécifique.

6. Procédé selon la revendication 2, dans lequel l'analyte a été isolé à partir de matériel naturel, par exemple animal ou humain, ou a été produit par synthèse ou génie génétique.

7. Procédé selon la revendication 2, dans lequel l'analyte ne correspond qu'en partie à l'analyte recherché ou a été modifié chimiquement ou biochimiquement

8. Procédé selon la revendication 3, dans lequel le partenaire de liaison à affinité est choisi dans le groupe qui comprend les substances suivantes anti-corps, lectine, avidine, streptavidine, biotine ou ses dérivés, facteur de complément C1, protéine se liant au mannane, cofacteur.

9. Procédé selon la revendication 1, dans lequel, pour effectuer le marquage détectable, on utilise un élément détectable radiochimiquement, un composé fluorescent ou chimiluminescent, une enzyme ou un cofacteur, qui peuvent être détectés dans une réaction ultérieure appropriée.

10. Procédé selon la revendication 9, dans lequel on utilise comme marqueur une enzyme, de préférence la phosphatase alcaline ou la peroxydase de raifort.

11. Procédé selon la revendication 1, dans lequel la formation de complexes immuns est accélérée par l'addition de sulfate de dextrane et/ou de polyéthylèneglycol.

12. Procédé selon la revendication 2, dans lequel la substance de précipitation est dirigée contre l'analyte.

13. Procédé selon la revendication 12, dans lequel le conjugué consiste en un analyte modifié marqué qui ne réagit pas avec la substance de précipitation.

14. Procédé selon la revendication 1, dans lequel la substance de précipitation est dirigée contre une substance couplée additionnellement au premier partenaire de liaison spécifique non marqué.

15. Procédé selon l'une quelconque des revendications 1 à 14, dans lequel, pour l'accentuation de la vitesse de sédimentation, la substance de précipitation est elle-même immobilisée sur une phase solide insoluble.

16. Procédé selon la revendication 15, dans lequel le premier partenaire de liaison spécifique non marqué n'est pas fixé à une phase solide.

17. Procédé selon la revendication 15, dans lequel la phase sollde utilisée pour l'immobilisation est choisie dans le groupe des particules connues en soi de l'homme du métier, telles que : verre, gélatine, agarose, lipides, érythrocytes, plaquettes sanguines, leucocytes, colloïdes métalliques, matériaux synthétiques, qui sont de préférence magnétisables.

18. Procédé selon la revendication 17, dans lequel les particules synthétiques sont choisies dans le groupe constitué par le polystyrène, le polydextrane, le polypropylène, le poly(chlorure de vinyle), le poly(fluorure de vinylidène), le polyacrylamide ou des copolymères styrène/butadiène, styrène/acide méthacrylique ou méthacrylate/acide méthacrylique.

19. Procédé selon la revendication 1, dans lequel la substance de précipitation est un anticorps, la lectine, un substrat ou ses analogues, l'avidine, la streptavidine, la biotine ou ses dérivés, le facteur de complément C1, la protéine se liant au mannane, un cofacteur ou une autre substance qui entre en combinaison spécifique avec le partenaire de liaison désiré.

20. Procédé selon la revendication 18, dans lequel la substance de précipitation est un anticorps.

21. Procédé selon la revendication 1, dans lequel la réaction de précipitation (f) est accélérée par variation du milieu réactionnel.

22. Procédé selon la revendication 21, dans lequel l'accélération de la séparation est réalisée par une modification du pH.

23. Procédé selon la revendication 1, dans lequel on utilise des cellules en tant que phase solide particulaire apte au pipetage et on utilise comme substances de précipitation des anticorps dirigés contre un ou plusieurs antigènes de surface de ces cellules.

24. Procédé selon la revendication 1, dans lequel on utilise des particules magnétisables en tant que phase solide particulaire apte au pipetage et on utilise comme substances de précipitation des particules magnétiques, ou, dans le procédé inverse, on utilise des particules magnétiques en tant que phase solide et d'autres particules magnétiques ou magnétisables pour la précipitation.
